(19) Europäisches Patentamt
European Patent Office
Office européen des brevets

(11) Veröffentlichungsnummer: **0 027 580**
**B1**

(12) # EUROPÄISCHE PATENTSCHRIFT

(45) Veröffentlichungstag der Patentschrift: **23.02.83**

(51) Int. Cl.³: **C 07 C 93/197,** C 07 D 295/08, C 07 C 45/65

(21) Anmeldenummer: **80105961.9**

(22) Anmeldetag: **02.10.80**

(54) **Cyclohexanone und Cyclohexenone, Verfahren zu ihrer Herstellung und ihre Verwendung.**

(30) Priorität: **13.10.79 DE 2941625**

(43) Veröffentlichungstag der Anmeldung: **29.04.81 Patentblatt 81/17**

(45) Bekanntmachung des Hinweises auf die Patenterteilung: **23.02.83 Patentblatt 83/8**

(84) Benannte Vertragsstaaten: **CH DE FR GB LI**

(56) Entgegenhaltungen:
**FR-A-2 372 787**

**„Journal of Organic Chemistry", Bd. 35, Nr. 9, September 1970, S. 2913-2918, C.A. Kingsbury: „Structures and reactions of condensation products of benzaldehyde and acetoacetic ester"**
**„Chemical Abstracts", Bd. 83, Nr. 3, 21. Juli 1975, S. 497, Nr. 28112a, Columbus, Ohio, USA**
**„Chemical Abstracts", Bd. 84, Nr. 25, 21. Juni 1976, S. 529, Nr. 179733a, Columbus, Ohio, USA, P.M. McCurry, Jr. *et al.*: „Cyclenones IX: A convenient synthesis of 3-methyl-5-alkyl-4-carbethoxycy-clohex-2-enones"**

(73) Patentinhaber: **HOECHST AKTIENGESELLSCHAFT, Postfach 80 03 20, D-6230 Frankfurt/Main 80 (DE)**

(72) Erfinder: **Berthold, Rüdiger, Dr., Geierfeld 55, D-6232 Bad Soden am Taunus (DE)**
Erfinder: **Müller, Werner Heinrich, Dr., Taunusblick 5, D-6239 Eppstein/Taunus (DE)**

**EP 0 027 580 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Cyclohexanone und -hexenone, Verfahren zu ihrer Herstellung und ihre Verwendung

Aus der DE-OS Nr. 2654850 ist es bekannt, aus Acetessigestern und Aldehyden substituierte Cyclohexanone und -hexenone herzustellen, wobei diese Kondensationsreaktionen in Gegenwart katalytischer Mengen aliphatischer tertiärer Amine durchgeführt werden. Die zugesetzten Amine katalysieren hierbei auch die Wasserabspaltung aus den zunächst erhaltenen Cyclohexanolonen zum Cyclohexenon sowie die Verseifung der zur Ketogruppe α-ständigen Carbonsäureestergruppe, die dadurch leicht decarboxyliert. Für die Verseifung der zweiten Carbonsäureestergruppe ist es jedoch erforderlich, eine Säure als Katalysator zuzusetzen.

Gegenstand der Erfindung sind Verbindungen der Formel 1

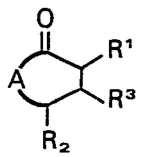

(1)

in welcher

A eine Gruppe der Formeln 1a oder 1b

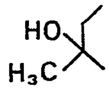 (1a) oder 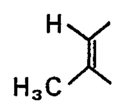 (1b)

ist, die die Formel 1 zu einem carbocyclischen Sechsring vervollständigt,

$R^1$ Wasserstoff oder eine Gruppe der Formel 1c

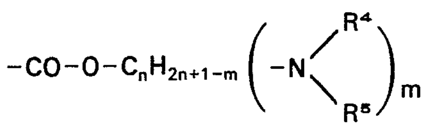

(1c)

$R^2$ eine Gruppe der Formel 1c,

$R^3$ Wasserstoff, Alkyl mit 1 bis 10 Kohlenstoffatomen oder unsubstituiertes oder durch niederes Alkyl, niederes Alkoxy, Trifluormethyl, Nitro, Cyan, Chlor oder Carbamoyl- oder Sulfamolyreste, die ihrerseits durch niederes Alkyl substituiert sein können, substituiertes Phenyl,

$R^4$ und $R^5$, die gleich oder verschieden sein können, niederes Alkyl bedeuten oder gemeinsam mit dem Stickstoffatom einen Pyrrolidin-, Piperidin- oder Morpholinring bilden,

m 1 oder 2, und

n 2 bis 6 bedeuten.

Niederes Alkyl bzw. Alkoxy bezeichnet hier und im folgenden Reste mit bis zu 4, vorzugsweise 1 oder 2 Kohlenstoffatomen.

Gegenstand der Erfindung ist weiterhin ein Verfahren zur Herstellung der Verbindungen der Formel 1, das dadurch gekennzeichnet ist, dass man 2 mol einer Verbindung der Formel 2

$$CH_3-CO-CH_2-CO-O-C_nH_{2n+1-m}(-NR^4R^5)_m \quad (2)$$

mit 1 mol eines Aldehyds der Formel 3

$$R^3-CHO \quad (3)$$

umsetzt.

Gegenstand der Erfindung ist weiterhin die Verwendung der Verbindungen der Formel 1 zur Herstellung von Verbindungen der Formel 4

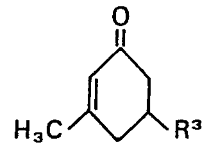

(4)

durch Verseifen und Decarboxylieren. Die Verbindungen der Formel 4 sind bekannt und dienen beispielsweise zur Herstellung der entsprechend substituierten Aniline über die Zwischenstufe der Cyclohexanonoxime (DE-OS Nrn. 2654851 und 2654852).

Gegenüber den aus der DE-OS Nr. 2654850 bekannten zeigen die erfindungsgemässen Verbindungen den Vorteil, dass auch die Verseifung und Decarboxylierung der zweiten Estergruppe ohne Zusatz eines Katalysators erfolgen kann. Es ist somit auch nicht erforderlich, den Katalysator abzutrennen, was die Isolierung des Produkts und vor allem auch des abgespaltenen Alkohols der Formel 5

$$HO-C_nH_{2n+1-m}(-NR^4R^5)_m \quad (5)$$

erleichtert. Dieser Alkohol kann somit leicht wiedergewonnen und, beispielsweise mit Diketen, in das Ausgangsmaterial der Formel 2 übergeführt werden. Auch für diese Umsetzung ist — im Gegensatz zu unsubstituierten Alkanolen — kein Katalysator erforderlich. Das erfindungsgemässe Verfahren eröffnet somit die Möglichkeit, die Verbindungen der Formel 4 aus 2 mol Diketen und 1 mol des Aldehyds der Formel 3 herzustellen, wobei keine Katalysatoren erforderlich sind und als Nebenprodukt nur Kohlendioxid anfällt.

Ein weiterer Vorteil gegenüber dem aus der DE-OS Nr. 2654850 bekannten Verfahren ist es, dass das erfindungsgemässe Verfahren leicht so durchgeführt werden kann, dass die Verbindungen der Formel 1 flüssig bleiben und somit leicht aus dem Reaktionsgefäss ausgetragen werden können. Da die Produkte katalysatorfrei sind und ohne weitere Zusätze in die Verbindungen der Formel 4 übergeführt werden können, können kostspielige Reinigungsoperationen entfallen.

Die Ausgangsmaterialien der Formel 2 sind bekannt und — wie vorstehend erwähnt — leicht aus Diketen und dem entsprechenden Alkohol erhältlich. Da für die erfindungsgemässe Verwendung der neuen Verbindungen die Natur des Alkohols 5 nicht von Belang ist, wählt man zweckmässig gut zugängliche und leicht wieder abtrennbare Alkohole 5. Bevorzugt sind dementsprechend Verbindungen, in denen m 1, und n 2 oder 3, bedeuten und $R^4$ und $R^5$ gleich sind und Methyl oder Ethyl bedeuten. Der Einsatz anderer Verbindungen der Formel 2 ist möglich, aber für die erfindungsgemässe Verwendung ohne Vorteil.

Bevorzugte Aldehyde der Formel 3 sind Alkanale mit bis zu 5 Kohlenstoffatomen, insbesondere

mit 2 bis 4 Kohlenstoffatomen. Von den aromatischen Aldehyden werden Benzaldehyd sowie durch Methyl, Methoxy und Chlor substituierte Benzaldehyde bevorzugt.

Die Umsetzung der Verbindung 2 mit dem Aldehyd 3 kann bei Temperaturen zwischen etwa −40 und 120°C durchgeführt werden. Bevorzugt sind Temperaturen zwischen etwa 0 und 80°C, insbesondere zwischen 20 und 70°C. Es ist möglich, das erfindungsgemässe Verfahren in Gegenwart eines inerten Löse- oder Verdünnungsmittels durchzuführen. Es ist auch möglich, den Ester 2 im Überschuss einzusetzen.

Selbstverständlich richten sich die Verfahrensbedingungen nach der Reaktivität der Ausgangsmaterialien und nach der Natur des gewünschten Verfahrensproduktes. Im allgemeinen werden die Primärprodukte, nämlich die Cyclohexanolone der Formel 1a, in denen $R^1$ die Gruppe 1c bedeutet, nicht isoliert, sondern unmittelbar der Wasserabspaltung zu den Verbindungen der Formel 1b unterworfen. Da auch die Abspaltung der benachbart zur Ketogruppe stehenden Carbonestergruppe leicht erfolgt, wird im allgemeinen das Produkt der Formel 1b erhalten, in dem $R^1$ Wasserstoff ist.

Für die erfindungsgemässe Verwendung der Verbindungen der Formel 1 ist es nicht erforderlich, diese zu isolieren. Kondensation und Verseifung/Decarboxylierung können somit in einer Eintopfreaktion erfolgen.

Für die Verseifung der zur Ketogruppe benachbarten Estergruppe genügt im allgemeinen das bei der Kondensation abgespaltene Wasser. Zur quantitativen Verseifung der zweiten Estergruppe wird dem Ansatz Wasser zugesetzt und die Reaktionsmischung erhitzt. Dieses Erhitzen kann im offenen Gefäss oder — zur Abkürzung der Reaktionsdauer — unter Druck erfolgen.

In den folgenden Beispielen wird die Erfindung näher erläutert. Prozentangaben beziehen sich auf das Gewicht, wenn nichts anderes angegeben ist.

*Beispiel 1*

Zu 173 g Acetessigsäure-2-dimethylaminoethylester (1,0 mol), Siedepunkt bei 2,66 mbar 106-107°C, gibt man langsam 22,5 g Acetaldehyd (0,51 mol), so dass die Temperatur 50°C nicht übersteigt. Bereits nach Zugabe von etwa 7 g Acetaldehyd innerhalb von 3 min steigt die Temperatur von 22 auf 42°C. Wenn der gesamte Acetaldehyd zugegeben ist, rührt man noch 20 min ohne Kühlung nach, erwärmt auf 80°C und rührt 5 h unter Rückfluss. Man erhält 173 g Produkt, entsprechend einem Verlust von etwa 22 g = 0,5 mol Kohlendioxid.

Das Produkt wird am absteigenden Kühler zuerst im Wasserstrahlvakuum bei einer Heizbadtemperatur von 100°C, dann im Ölpumpenvakuum (2,7 mbar), ebenfalls bei 100°C Badtemperatur, destilliert, wobei die niedrig siedenden Anteile (Wasser, Dimethylaminoethanol und wenig 3,5-Dimethylcyclohex-2-en-1-on) übergehen. Der Destillationsrückstand (106 g) besteht zu 98% aus 3,5-Dimethylcyclohex-2-en-1-on-4-carbonsäure-2'-dimethylaminoethylester und zu 2% aus höher siedenden Produkten. Danach beträgt die Ausbeute 104 g Rohester, 100%ig, entsprechend 87% der Theorie.

Dieser Rohester kann direkt weiterverarbeitet werden. Er lässt sich, wenn auch unter geringer Zersetzung, destillieren (Siedepunkt bei 2,66 mbar 162°C). Das gelbe Destillat hat einen Reingehalt von etwa 90%. Der Rest besteht aus tiefer siedenden Produkten (Dimethylaminoethanol, 3,5-Dimethylcyclohex-2-en-1-on), die sich bei einer Badtemperatur von etwa 100°C und einem Druck von etwa 2,7 mbar ohne Kolonne entfernen lassen. Der Destillationsrückstand beträgt 10% vom Einsatz.

*Beispiel 2*

Zu 173 g frisch destilliertem Acetessigsäure-2-dimethylaminoethylester (1,0 mol) gibt man auf einmal 36 g Butyraldehyd (0,5 mol). Dabei steigt die Temperatur innerhalb von 10 min auf 51°C an, beim weiteren Nachrühren nach 30 min auf 61°C. Man setzt 5 ml Wasser zu und rührt 1 h bei 80°C unter Rückfluss. Danach entfernt man bei 120°C Badtemperatur und einem Vakuum von 2,7 mbar die niedrig siedenden Anteile. Man erhält als Rückstand 120 g rohen 3-Methyl-5-propylcyclohex-2-en-1-on-4-carbonsäure-2'-dimethylaminoethylester, 91%ig, entsprechend 110 g 100%iges Produkt, oder 82,3% der Theorie. Als Destillat fallen 65 g einer farblosen Flüssigkeit an, die zu 81% aus Dimethylaminoethanol besteht (0,59 mol).

Der Rohester lässt sich nur in kleinen Portionen (etwa 30-50 g) destillieren. Der Siedepunkt bei 1,5 mbar liegt bei 170-172°C, der Reingehalt beträgt 96%.

Es verbleiben etwa 10% vom Einsatz als Rückstand. Grössere Mengen können über eine Dünnschichtdestillieranlage destilliert werden.

*Beispiel 3*

Zu 183 g frisch destilliertem Acetessigsäure-2-dimethylaminoethylester (1,0 mol) gibt man auf einmal 53 g (0,5 mol) Benzaldehyd. Innerhalb von 10 min steigt die Temperatur auf 40°C und fällt dann allmählich wieder ab. Beim Rühren über Nacht verwandelt sich die Reaktionsmischung in einen Kristallbrei. Nach Absaugen erhält man 50 g 3-Phenyl-5-methylcyclohexan-5-ol-1-on-2,4-di(carbonsäure-2'-dimethylaminoethylester) in Form fettiger Kristalle vom Schmelzpunkt 123-124°C, entsprechend 26% der Theorie, sowie 170 g eines Öls.

Das Öl wird 6 h auf 80°C erwärmt und anschliessend die tiefsiedenden Anteile, wie in Beispiele 1 und 2 beschrieben, abgetrennt. Man erhält 98 g 3-Methyl-5-phenylcyclohex-2-en-1-on-4-carbonsäure-2'-dimethylaminoethylester mit einem Reingehalt von 90%, entsprechend 88 g 100%iges Produkt oder 58,7% der Theorie. Dieser Rohester lässt sich im Ölpumpenvakuum nicht destillieren.

Wird das vorstehend genannte Öl einige Tage im Kühlschrank aufbewahrt, so lässt sich eine zweite kristalline Fraktion gewinnen.

*Beispiel 4*

Zu 95 g frisch destilliertem Acetessigsäure-1-dimethylamino-2-propylester (Siedepunkt bei 0,95 mbar 74-75°C), 98%ig (0,5 mol), gibt man auf einmal 12 g Acetaldehyd (0,25 mol). Innerhalb von 5 min steigt die Temperatur von 19 auf 50°C an. Es wird ½ h nachgerührt und dann 5 h auf 80°C erwärmt. Bei einer Badtemperatur von 110°C werden die tiefsiedenden Anteile bei einem Druck von maximal 2,7 mbar abgetrennt. Man erhält 61,3 g rohen 3,5-Dimethylcyclohex-2-en-1-on-4-carbonsäure-1'-dimethylamino-2'-propylester mit einem Reingehalt von 98%, entsprechend 60 g 100%iges Produkt oder 95% der Theorie. Der Ester lässt sich gut destillieren (Siedepunkt bei 2,7 mbar 156-157°C), wobei 82% der Theorie an 100%igem Produkt erhalten werden.

*Beispiel 5*

Zu 346 g frisch destilliertem Acetessigsäure-2-dimethylaminoethylester (2,0 mol) gibt man langsam 44 g frischen Acetaldehyd (1,0 mol). Die Mischung wird 1 h bei Raumtemperatur und anschliessend 5 h bei 80°C gerührt. Bei einer Badtemperatur von 100°C werden im Vakuum Dimethylaminoethanol und etwas Wasser abdestilliert. Es werden 115 g Destillat erhalten, das zu 15% (17,3 g) aus Wasser und zu 85% (97,8 g) aus Dimethylaminoethanol besteht. Als Rückstand verbleiben 230 g eines Gemisches aus 2,5% 3,5-Dimethylcyclohex-2-en-1-on, 1,5% Dimethylaminoethanol und 96% 3,5-Dimethylcyclohex-2-en-1-on-4-carbonsäure-2'-dimethylaminoethylester. Diesen Rückstand und 230 g Wasser oder Destillat aus einem vorhergehenden Ansatz erhitzt man in einem Autoklav 5 h auf 140°C. Nach Destillieren an einer Kolonne erhält man zunächst bei einem Siedepunkt bis 102°C eine Fraktion von 285 g (davon 10 g 3,5-Dimethylcyclohex-2-en-1-on, das mit dem Wasserdampf übergegangen ist und abgetrennt wird). Diese wässerige ketonhaltige Phase wird bei der nächsten Verseifung wieder eingesetzt. Als weitere Fraktionen werden erhalten: im Siedebereich von 102-135°C 52 g Dimethylaminoethanol und im Siedebereich von 93-110°C bei 24 mbar 89 g 3,5-Dimethylcyclohex-2-en-1-on, also insgesamt 99 g 3,5-Dimethylcyclohex-2-en-1-on oder 79,8% der Theorie.

*Beispiel 6*

Zu 72,5 g Acetessigsäure-1,3-di(dimethylamino)-2-propylester, 92%ig, gibt man 6,5 g Acetaldehyd. Die Temperatur steigt von 26 auf 53°C an. Man rührt zunächst 1 h ohne Erwärmung, dann 5 h bei 80°C nach. Anschliessend wird das Reaktionsgemisch im Vakuum destilliert. Hierbei gehen als Vorlauf 20 g einer Fraktion im Siedebereich 49-172°C bei 1,1 bis 2 mbar über, bestehend aus Wasser und 1,3-Di(dimethylamino)-2-propanol. Der Hauptlauf besteht aus 3,5-Dimethylcyclohex-2-en-1-on-4-carbonsäure-1',3'-di-(dimethylamino)-2'-propylester vom Siedepunkt 177-178°C bei 2,66 mbar. Nach Destillieren, wobei sich ein wenig des Esters zersetzt, erhält man 29 g Reinprodukt oder 67% der Theorie.

*Beispiel 7*

Analog Beispiel 6 erhält man aus Acetessigsäure-1-diethylamino-2-propylester und Acetaldehyd 3,5-Dimethylcyclohex-2-en-1-on-4-carbonsäure-1'-diethylamino-2'-propylester vom Siedepunkt 168-170°C bei 1,6 mbar; Ausbeute 72% der Theorie.

*Beispiel 8*

Analog Beispiel 6 erhält man aus Acetessigsäure-1-piperidyl-2-propylester und Acetaldehyd 3,5-Dimethylcyclohex-2-en-1-on-4-carbonsäure-1'-piperidyl-2'-propylester vom Siedepunkt 187-189°C bei 2,66 mbar (Zersetzung). Die Ausbeute beträgt 50% der Theorie (gemäss gaschromatographischer Untersuchung liegt die Rohausbeute vor der Destillation bei 80-90% der Theorie).

**Patentansprüche**

1. Verbindungen der Formel (1)

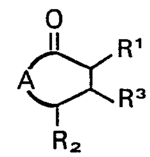

(1)

in welcher

A eine Gruppe der Formeln (1a) oder (1b)

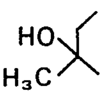 (1a) oder 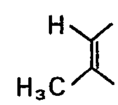 (1b)

ist, die die Formel (1) zu einem carbocyclischen Sechsring vervollständigt,

$R^1$ Wasserstoff oder eine Gruppe der Formel (1c)

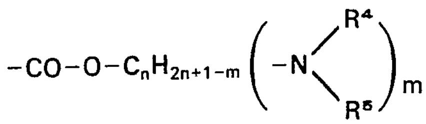

(1c)

$R^2$ eine Gruppe der Formel (1c),

$R^3$ Wasserstoff, Alkyl mit 1 bis 10 Kohlenstoffatomen oder unsubstituiertes oder durch niederes Alkyl, niederes Alkoxy, Trifluormethyl, Nitro, Cyan, Chlor oder Carbamoyl- oder Sulfamoylreste, die ihrerseits durch niederes Alkyl substituiert sein können, substituiertes Phenyl,

$R^4$ und $R^5$, die gleich oder verschieden sein können, niederes Alkyl bedeuten oder gemeinsam mit dem Stickstoffatom einen Pyrrolidin-, Piperidin- oder Morpholinring bilden,

m 1 oder 2,

n 2 bis 6, und

niederes Alkyl bzw. Alkoxy Reste mit bis zu 4, vorzugsweise 1 oder 2, Kohlenstoffatomen bedeuten.

2. Verbindungen nach Anspruch 1, in denen m 1 und 2 oder 3, $R^4$ und $R^5$ Methyl oder Ethyl oder $-NR^4R^5$ Piperidyl bedeuten.

3. Verbindungen nach den beiden Ansprüchen 1 und 2, in denen $R^3$ Wasserstoff oder Alkyl mit 1 bis 4 Kohlenstoffatomen, Phenyl oder durch Methyl, Methoxy und Chlor substituiertes Phenyl ist.

4. Verbindungen nach den Ansprüchen 1 bis 3, in denen A eine Gruppe der Formel (1a) und $R^1$ eine Gruppe der Formel (1c) ist.

5. Verbindungen nach den Ansprüchen 1 bis 3, in denen A eine Gruppe der Formel (1b) und $R^1$ Wasserstoff ist.

6. Verfahren zur Herstellung der Verbindungen nach Anspruch 1, dadurch gekennzeichnet, dass man 2 mol einer Verbindung der Formel (2)

$$CH_3-CO-CH_2-CO-O-C_nH_{2n+1-m}(-NR^4R^5)_m \quad (2)$$

mit 1 mol eines Aldehyds der Formel (3)

$$R^3-CHO \quad (3)$$

umsetzt, wobei $R^3$, $R^4$, $R^5$, m und n die in Anspruch 1 genannte Bedeutung haben.

7. Verfahren nach Anspruch 6, dadurch gekennzeichnet, dass die Umsetzung bei −40 bis +120°C durchgeführt wird.

8. Verfahren nach den beiden Ansprüchen 6 und 7, dadurch gekennzeichnet, dass die Umsetzung bei 0 bis 80°C durchgeführt wird.

9. Verfahren nach den Ansprüchen 6 bis 8, dadurch gekennzeichnet, dass die Umsetzung bei 20 bis 70°C durchgeführt wird.

10. Verwendung der Verbindungen nach Anspruch 1 zur Herstellung von Verbindungen der Formel (4)

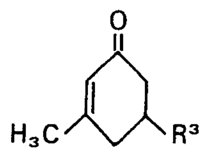

(4)

in der $R^3$ die in Anspruch 1 genannte Bedeutung hat, durch Verseifen und Decarboxylieren.

## Claims

1. Compounds of the formula (1)

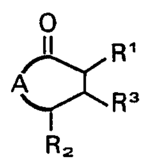

(1)

in which A is a group of the formula (1a) or (1b)

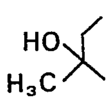

(1a)

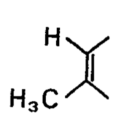

(1b)

which complete formula (1) to a carboxylic 6-membered ring,

$R^1$ is hydrogen or a group of the formula (1c)

$-CO-O-C_nH_{2n+1-m}$ 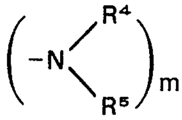 (1c)

$R^2$ is a group of the formula (1c),

$R^3$ is hydrogen, alkyl of 1 to 10 carbon atoms, unsubstituted phenyl, phenyl substituted by lower alkyl, lower alkoxy, trifluoromethyl, nitro, cyano, chloro, carbamoyl or sulfamoyl which may be substituted themselves by lower alkyl,

$R^4$ and $R^5$, which are the same or different, are lower alkyl or, together with the nitrogen atom, form a pyrrolidine, piperidine or morpholine ring,

m is 1 or 2,

n is 2 to 6, and

lower alkyl and alkoxy are groups with up to 4, preferably 1 or 2, carbon atoms.

2. Compounds as claimed in claim 1, wherein m is 1, n is 2 or 3 and $R^4$ and $R^5$ are methyl or ethyl or $-NR^4R^5$ is piperidyl.

3. Compounds as claimed in claims 1 and 2, wherein $R^3$ is hydrogen, alkyl of 1 to 4 carbon atoms, phenyl or phenyl substituted by methyl, methoxy and chlorine.

4. Compounds as claimed in claims 1 to 3, wherein A is a group of the formula (1a) and $R^1$ is a group of the formula (1c).

5. Compounds as claimed in claims 1 to 3, wherein A is a group of the formula (1b) and $R^1$ is hydrogen.

6. A process for the preparation of compounds as defined in claim 1, characterized by reacting 2 mol of an ester of the formula (2)

$$H_3C-CO-CH_2-CO-O-C_nH_{2n+1-m}(-NR^4R^5)_m \quad (2)$$

with 1 mol of an aldehyde of the formula (3)

$$R^3-CHO \quad (3)$$

wherein $R^3$, $R^4$, $R^5$, n and m are defined as in claim 1.

7. A process as claimed in claim 6, characterized by carrying out the reaction at −40 to +120°C.

8. A process as claimed in claims 6 and 7, characterized by carrying out the reaction at 0 to 80°C.

9. A process as claimed in claims 6 to 8, characterized by carrying out the reaction at 20 to 70°C.

10. Use of the compounds as claimed in claim 1 for preparing compounds of the formula (4)

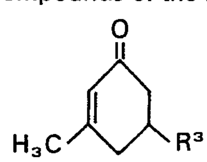

(4)

wherein $R^3$ is defined as in claim 1, by saponification and decarboxylation.

## Revendications

1. Composés caractérisés en ce qu'ils répondent à la formule

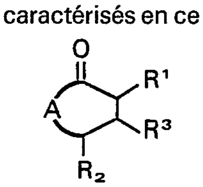

(1)

dans laquelle

A représente un groupe répondant à l'une des formules (1a) ou (1b)

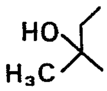 (1a) ou 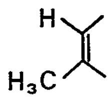 (1b)

qui complète la formule (1) pour l'obtention d'un noyau hexagonal carboxyclique,

$R^1$ est un atome d'hydrogène ou un groupe de formule (1c)

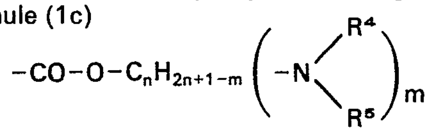 (1c)

$R^2$ est un groupe de formule (1c),

$R^3$ est un atome d'hydrogène, un groupe alkyle ayant 1 à 10 atomes de carbone ou un groupe phényle non substitué ou substitué par un groupe alkyle inférieur, alcoxy inférieur, trifluorométhyle, nitro, cyano, chloro ou carbamoyle ou sulfamoyle, qui peuvent être substitués à leur tour par un groupe alkyle inférieur,

$R^4$ et $R^5$, qui peuvent être identiques ou différents, représentent chacun un groupe alkyle inférieur ou bien ils forment, avec l'atome d'azote, un noyau pyrrolidine, pipéridine ou morpholine,

m vaut 1 ou 2,

n vaut 2 à 6, et

un groupe alkyle ou alcoxy inférieur désigne des radicaux ayant jusqu'à 4, et de préférence 1 ou 2 atomes de carbone.

2. Composés selon la revendication 1, caractérisés en ce que m vaut 1, n vaut 2 ou 3, $R^4$ et $R^5$ présentent chacun un groupe méthyle ou éthyle ou bien $-NR^4R^5$ est un noyau pipéridyle.

3. Composés selon les revendications 1 et 2, caractérisés en ce que $R^3$ représente un atome d'hydrogène ou un groupe alkyle ayant 1 à 4 atomes de carbone, phényle ou phényle substitué par un groupe méthyle, méthoxy ou chloro.

4. Composés selon l'une des revendications 1 à 3, caractérisés en ce que A est un groupe de formule (1a) et $R^1$ est un groupe de formule (1c).

5. Composés selon l'une quelconque des revendications 1 à 3, caractérisés en ce que A est un groupe de formule (1b) et $R^1$ est un atome d'hydrogène.

6. Procédé pour préparer les composés selon la revendication 1, caractérisé en ce qu'on fait réagir 2 mol d'un composé de formule (2)

$$CH_3-CO-CH_2-CO-O-C_nH_{2n+1-m}(-NR^4R^5)_m \quad (2)$$

avec 1 mol d'un aldéhyde de formule (3)

$$R^3-CHO \quad (3)$$

les symboles $R^3$, $R^4$, $R^5$, m et n ayant le sens indiqué à la revendication 1.

7. Procédé selon la revendication 6, caractérisé en ce qu'on conduit la réaction entre −40° et +120°C.

8. Procédé selon les revendications 6 et 7, caractérisé en ce qu'on conduit la réaction entre 0 et 80°C.

9. Procédé selon l'une quelconque des revendications 6 à 8, caractérisé en ce qu'on conduit la réaction entre 20 et 70°C.

10. Utilisation des composés selon la revendication 1 pour la préparation, par saponification et décarboxylation, de composés de formule (4)

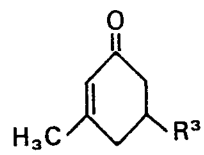 (4)

dans laquelle $R^3$ a le sens indiqué à la revendication 1.